## Europäisches Patentamt
## European Patent Office
## Office européen des brevets

(19)

(11) Veröffentlichungsnummer: **0 020 905**
**B1**

(12)

# EUROPÄISCHE PATENTSCHRIFT

(45) Veröffentlichungstag der Patentschrift:
18.07.84

(51) Int. Cl.³: **A 61 K 9/70**, A 61 L 15/03

(21) Anmeldenummer: 80102015.7

(22) Anmeldetag: 15.04.80

(54) Pharmazeutische Zubereitung in Form eines Polyacrylatfilmes und deren Herstellung.

(30) Priorität: 21.05.79 DE 2920500

(43) Veröffentlichungstag der Anmeldung:
07.01.81 Patentblatt 81/1

(45) Bekanntmachung des Hinweises auf die Patenterteilung:
18.07.84 Patentblatt 84/29

(84) Benannte Vertragsstaaten:
AT BE CH DE FR GB IT LI LU NL SE

(56) Entgegenhaltungen:
DE - A - 2 006 696
DE - A - 2 214 306
FR - A - 2 150 084
FR - A - 2 212 134
FR - A - 2 397 190
US - A - 3 579 628
US - A - 3 598 123
US - A - 3 699 963
US - A - 4 076 798

Die Akte enthält technische Angaben, die nach dem Eingang der Anmeldung eingereicht wurden und die nicht in dieser Patentschrift enthalten sind.

(73) Patentinhaber: **C.H. BOEHRINGER SOHN, Postfach 200, D-6507 Ingelheim am Rhein (DE)**

(72) Erfinder: **Stricker, Herbert, Dr., Stettiner Strasse 2, D-6507 Ingelheim/Rhein (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft eine pharmazeutische Zubereitung in Form eines Polyacrylatfilms, die für die transdermale Langzeittherapie mit systemisch wirksamen Arzneistoffen verwendbar ist, sowie deren Herstellung.

Viele Arzneistoffe sind befähigt, die Haut zu durchdringen und in den allgemeinen Kreislauf zu gelangen, wo sie systemisch zur Wirkung kommen. Arzneistoffe, die niedrig dosiert werden können und deren Penetrationsfähigkeit so groß ist, daß die Geschwindigkeit der Invasion in den allgemeinen Kreislauf über die Geschwindigkeit der Wirkstofffreisetzung aus der Zubereitung gesteuert werden kann, lassen sich mit Hilfe der beschriebenen Darreichungsform über längere Zeit zuverlässiger und genauer verabreichen als z. B. per os. Die Vorteile des Arzneistofftransportes in den allgemeinen Kreislauf durch die Haut beruhen vor allem auf der Ausschaltung nicht beeinflußbarer Faktoren der gastrointestinalen Resorption, der Verminderung des Metabolismus durch Vermeidung der ersten Leberpassage und auf den Effekten, die ganz allgemein bei Retardform angestrebt werden, wie z. B. Vermeidung hoher Blutspiegelanfangskonzentrationen und Erreichung konstanter Blutspiegel über längere Zeit.

Es sind bisher verschiedene transdermale Freigabesysteme mit systemisch wirksamen Arzneistoffen bekanntgeworden. Die US-A-3 598 122, 3 598 123, 3 742 951, 3 797 494, 3 996 934, 3 995 632, 4 060 084, 3 731 683 und 4 031 894 beschreiben sandwichartige bzw. laminar aufgebaute Bandagen und Heftpflaster, die aus einem Stützgerüst, welches die eine Oberfläche der Bandage bildet, einem Reservoir, welches einen systemisch aktiven Wirkstoff in fester suspendierter Form enthält, aus einer die Oberfläche des Reservoirs aufgebrachte Vorrichtung zur Regelung der Freigabegeschwindigkeit sowie aus Mitteln zum Befestigen der Bandage bzw. des Pflasters an der Haut bestehen. Daneben sind aus den DE-A-2 432 925, 2 218 200 und 2 207 635 Arzneistoffträger in Folienform mit inkorporierten Wirkstoffen bekannt, die aus Hydroxyalkylcellulose bzw. Polyaminosäuren etc. bestehen.

Die mehrschichtig zusammengesetzten Systeme sind aufgrund ihres komplizierten Aufbaus schwierig herzustellen und erfordern einen hohen Investitions- und Herstellungskostenaufwand. Darüber hinaus wird der in den mehrschichtigen Systemen in suspendierter Form enthaltende Wirkstoff nur in begrenztem Maße ausgenutzt, was einer geringen Bioverfügbarkeit entspricht. Die übrigen polymeren Wirkstoffträger bestehen aus Stoffen, die in Wasser löslich und daher für eine dermale Langzeitapplikation nicht geeignet sind.

Die DE-A-2 006 696 offenbart einen Haftverband, der aus einem Trägerteil in Form eines Kunststofffilms und einer Klebeschicht besteht. Der Wirkstoff ist entweder in die Klebmittelschicht eingearbeitet oder in einem Hohlraum des Pflasters in Form einer Tablette, einer Salbe, eines Pulvers usw. untergebracht. Als Klebmittel werden verschiedene Stoffe, u. a. Polyvinylbutyläther oder Acrylat- oder Methacrylatpolymerisate, vorgeschlagen. Die in der Druckschrift vermittelte Lehre hat nicht zum Ziel, eine konstante, reproduzierbare Freigabe des Wirkstoffes zu gewährleisten. Es läßt sich lediglich entnehmen, daß ausreichende Mengen des Wirkstoffes durch die Haut systemisch aufgenommen werden sollen, um die dort beschriebenen Wirkungen hervorzurufen.

Die DE-A-2 214 306 betrifft ein gegebenenfalls wirkstoffhaltiges Wundfilm-Spray, das nach Aufsprühen auf die Haut einen Polymerfilm hinterläßt. Als filmbildende Polymerisate werden u. a. Polyacrylsäure- und Polymethacrylsäureester vorgeschlagen. Das dort beschriebene Mittel kann auch als hautabdeckendes Mittel für ungeschädigte Haut als Schutzfilm verwendet werden.

In der Veröffentlichung findet sich jedoch nicht der Vorschlag, Wirkstoffe mit kontrollierter Freigabegeschwindigkeit systemisch durch die Haut zu applizieren. Ein solches Mittel ist nicht geeignet, eine gut reproduzierbare Dosierung des Wirkstoffes zu gewährleisten, denn in Abhängigkeit von der Sprühdauer, Sprühabstand und Krümmung der Hautoberfläche werden unterschiedliche, nicht reproduzierbare Filmflächen und -dicken erzeugt.

Der Gegenstand der FR-A-2 212 134 ist mit dem der DE-A-2 214 306 verwandt. Auch hier handelt es sich um einen flüssigen Wundverband zur Abdeckung beschädigter Hautbezirke. Soweit das Mittel Wirkstoffe enthält, dienen diese zur topischen, jedoch nicht zur systemischen Anwendung. Im übrigen gelten die gleichen Überlegungen, wie sie bei der Würdigung der DE-A-2 214 306 vorgebracht wurden.

Die US-A-4 076 798 schließlich betrifft eine pharmazeutische Zubereitung zur geregelten kontinuierlichen Applikation einer vorherbestimmten Wirkstoffmenge. Als Träger der pharmazeutischen Zubereitung wird ein biologisch abbaubares, hydrolysierbares Polyesterharz der Diglykolsäure vorgeschlagen.

Ziel der vorliegenden Erfindung ist es, eine pharmazeutische Zubereitung in Form eines Filmes bereitzustellen, der sich einfach und kostengünstig herstellen läßt, der eine gut reproduzierbare, kontrollierte Freigabe mit geringer Temperaturabhängigkeit und konstanter Geschwindigkeit bis zu hohen Umsätzen gewährleistet und der eine Variation der Freigabegeschwindigkeit in weiten Grenzen ermöglicht.

Erfindungsgemäß wird eine pharmazeutische, für die transdermale Applikation von Wirkstoffen geeignete Zubereitung in Form eines Filmes aus einem Copolymerisat von Methyl- und/oder Äthylestern der Acryl- und Methacrylsäure vorgeschlagen, die dadurch gekennzeichnet ist, daß der Wirkstoff im Polymerfilm in amorpher Form bzw. als feste Lösung in ansteigender Konzentration mit zunehmender Entfernung von der Freigabefläche vorliegt.

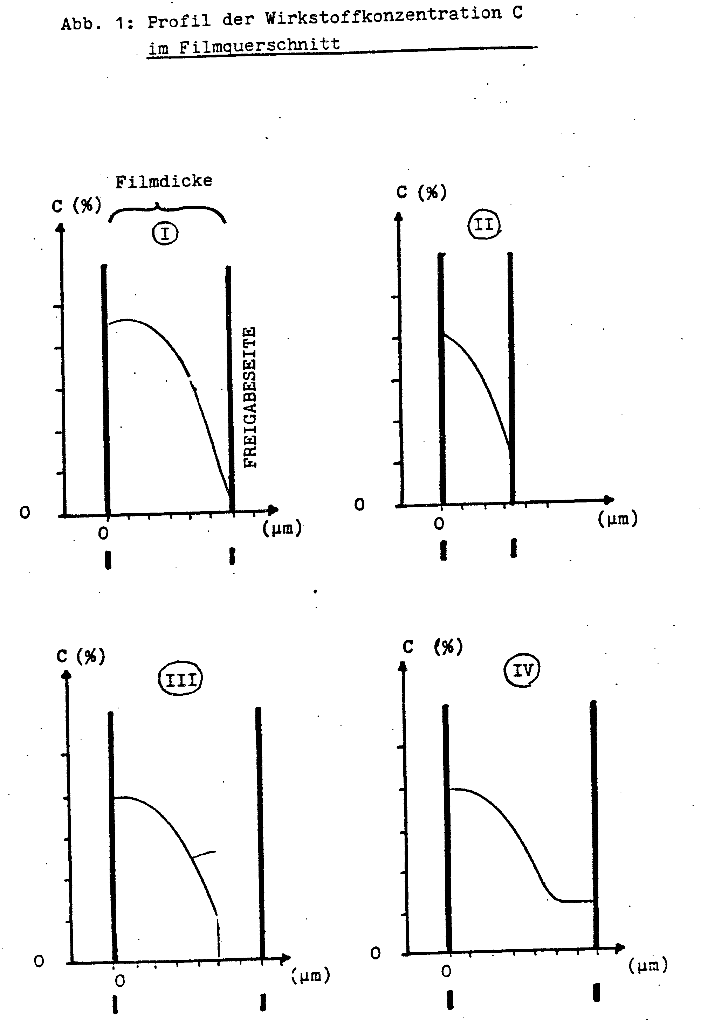

Bisher wurde nach dem Stand der Technik versucht, die Abnahme der Freigabegeschwindigkeit, die durch die Zunahme der Diffusionsschichtdicke verursacht wird, durch ein Depot an festem Arzneistoff und eine gesättigte Lösung in der Filmmatrix zu verhindern. Die erfindungsgemäße Lösung der Aufgabe, eine konstante Freisetzungsgeschwindigkeit zu erzielen, besteht demgegenüber darin, die mit der Zeit zunehmende Diffusionsschichtdicke durch einen entsprechenden Gradienten der Wirkstoffkonzentration zu kompensieren, wie Abb. 1 zeigt, so daß die Freigabegeschwindigkeit bis zu relativ hohen Freigaberaten nahezu konstant bleibt. Die Lösung des Problems wird durch den Stand der Technik weder gelehrt noch nahegelegt.

Ein für die Herstellung des Filmes besonders geeignetes Ausgangsmaterial ist eine wäßrige Dispersion des Polyacrylats (PA), wie z. B. das auf dem Markt erhältliche Produkt Eudragit E 30 D oder Plex 4791 D der Fa. Röhm, Darmstadt. Der Film kann zur Regulierung der Freigabegeschwindigkeit des Arzneistoffes hydrophile Hilfsstoffe enthalten, wie Polyäthylenglycol, Glycerin, Sorbit etc.

Die Herstellung der erfindungsgemäßen pharmazeutischen Zubereitung erfolgt in der Weise, daß man in der wäßrigen Polyacrylatdispersion zunächst gegebenenfalls die obengenannten Hilfsstoffe und — sofern es sich um sehr gut wasserlösliche Arzneistoffe handelt — diese in der gewünschten Menge löst. Die Dispersion wird dann auf eine plane begrenzte Fläche ausgegossen und eventuell bei erhöhter Temperatur trocknen gelassen. Anschließend werden die Arzneistoffe in Form einer organischen Lösung (z. B. Äthanol, Äthanol-Wasser-Gemischen, Methylenchlorid oder als Suspension in Frigen bzw. Wasser) auf den trockenen Polyacrylatfilm aufgetragen und das Lösungsmittel verdampft.

Erforderlichenfalls kann auf diesen Film, der nach entsprechender Konfektionierung die fertige pharmazeutische Zubereitungsform im Sinne der Erfindung bereits darstellt, zur Variation des Wirkstoff-Konzentrationsprofils über den Filmquerschnitt auf der einen oder auf beiden Seiten des hergestellten Filmes erneut einmal oder mehrmals Dispersion aufgetragen und mit Wirkstoff beladen werden. In allen Fällen verbindet sich das nachträglich aufgetragene Polyacrylat mit dem Untergrund derart, daß ein völlig homogener Film resultiert.

Als besonders wichtige Faktoren, die die Freigabegeschwindigkeit beeinflussen, haben sich neben der Art des Filmbildners herausgestellt:

(a)  die Bedingungen, unter denen der Polyacrylatfilm mit Wirkstoff beladen wird (Art und Menge des Lösungsmittels, Temperatur etc.),
(b)  die Filmdicke sowie
(c)  Art und Menge der der Polyacrylatdispersion zugesetzten wasserbindenden Hilfsstoffe.

Das für die Höhe und den Verlauf der Freigabegeschwindigkeit entscheidende Arzneistoffkonzentrationsprofil (s. Abb. 1) wird durch diese Faktoren in unterschiedlicher Weise beeinflußt. So verschiebt sich z. B. bei erhöhter Trocknungstemperatur und mit abnehmender Filmdicke das Profil I nach Profil II. Wasserbindende Hilfsstoffe, wie z. B. Polyäthylenglycol 400, führen einerseits zu einer Erhöhung der Wasseraufnahme des Filmes auf der Haut, andererseits zu einer signifikanten Verminderung der Temperaturabhängigkeit der Wirkstofffreigabe.

Als Arzneistoffe für die erfindungsgemäße pharmazeutische Zubereitung kommen z. B. in Frage:

Antihypertonica (z. B. Clonidin), Psychopharmaka (z. B. Haloperidol),
Coronarmittel (z. B. Nitroglycerin), Migränemittel (z. B. Dihydroergotamin),
Corticoide, Antikonzeptiva, Analgetica, Antirheumatica, Anticholenergica.

Die wirkstoffhaltigen Polyacrylatfilme können mit oder ohne eine spezielle Klebeschicht auf die Haut aufgebracht werden, da sie von Natur aus bereits gewisse Hafteigenschaften besitzen. Ohne spezielle Klebeschicht kann die Fixierung entweder mit Hilfe geeigneter Heftpflaster oder an den Extremitäten z. B. mit einem elastischen Band erfolgen. Die Haftung des Filmes wird stark erhöht, wenn die betreffende Hautpartie zuvor mit Äthanol befeuchtet wurde. Zur Auftragung einer Klebeschicht wird diese in der beschriebenen Weise auf den wirkstoffhaltigen Film aufgebracht, wobei jedoch statt PA z. B. eine dafür geeignete Acrylharzdispersion (z. B. Plex 4853 D, Fa. Röhm) verwendet wird.

Die Vorteile der erfindungsgemäßen pharmazeutischen Zubereitung bestehen darin, daß ein gut hautverträglicher, physiologisch unbedenklicher Filmbildner verwendet wird, in dem der Arzneistoff in amorpher Form (meist als feste Lösung) mit einem ganz bestimmten Profil verteilt ist. Weitere Vorteile sind:

Die Zubereitung läßt sich einfach, kostengünstig und ohne großen apparativen Aufwand (nur geringe Mengen organischer Lösungsmittel erforderlich) herstellen. Die Zubereitung weist eine gut reproduzierbare kontrollierte Freigabe mit konstanter Geschwindigkeit bis zu hohen Umsätzen und relativ geringer Temperaturabhängigkeit auf, so daß eine hohe Bioverfügbarkeit gewährleistet ist. Die Herstellung kann unterschiedlichen Bedingungen und Zielen angepaßt werden, insbesondere lassen sich verschiedene Freigabegeschwindigkeiten einstellen.

## Beispiel 1

### Clonidinhaltige Zubereitung

Die wäßrige Polyacrylatdispersion wird unter staubfreien Bedingungen auf eine glatte, sehr genau ausgelotete begrenzte Fläche (Platte aus Polypropylen) ausgegossen. Die Trocknung der Dispersion geschieht bei 22° C und 35% rel. Luftfeuchte. Nachdem die Dispersion zu einem klaren, transparenten, blasenfreien Film ausgetrocknet ist, wird die Wirkstofflösung aufgetragen und das Lösungsmittel unter den obengenannten Klimabedingungen durch Trocknung vollständig wieder entfernt. Anschließend wird erneut Dispersion aufgetragen und trocknen gelassen. Nach vollständiger Trocknung resultiert ein klarer, durchsichtiger, homogener Polyacrylatfilm. Eventuelle Hilfsstoffe werden zuvor der Polyacrylatdispersion zugesetzt. Die Zusammensetzung verschiedener Filme mit eingebettetem Clonidin ist in Tabelle 1 beschrieben.

## Beispiel 2

### Nitroglycerinhaltige Zubereitung

Pro $cm^2$ werden 25,5 mg Polyacrylat in Form einer 30%igen wäßrigen Dispersion wie in Beispiel 1 beschrieben zu einem Film verarbeitet, wobei anschließend durch Behandlung mit Methanol eine Glättung des Filmes vorgenommen wird. Die Beladung des Filmes mit Nitroglycerin (2,6 mg/$cm^2$) erfolgt durch 2malige Auftragung einer 1%igen äthanolischen Lösung und jeweiliger 24stündiger Trocknung. Zur Einstellung des optimalen Wirkstoffprofils werden abschließend auf beschriebene Weise nochmals 15,3 mg Polyacrylat/$cm^2$ aufgetragen.

## Beispiel 3

### Dihydroergotaminmethansulfonathaltige Zubereitung

Wie in Beispiel 1 beschrieben, wird der Polyacrylatfilm (15,4 mg/$cm^2$) mit 2,4 mg Wirkstoff/$cm^2$ in Form einer 2%igen Methanol-Chloroform-Lösung (a) bzw. Methanol-Lösung (b) beladen.

## Beispiel 4

### Haloperidolmethansulfonathaltige Zubereitung

Wie in Beispiel 1 beschrieben, wird ein Polyacrylatfilm (19,8 mg/$cm^2$) hergestellt und mit 6,15 mg Wirkstoff/$cm^2$ in Form einer Methanol-Chloroform-Lösung beladen. Nach Trocknung folgt eine weitere Auftragung von 8,1 mg PA/$cm^2$.

### Eigenschaften der wirkstoffhaltigen Polymerfilme

Zur Bestimmung der in vitro-Wirkstofffreigabe wurden ca. 5 $cm^2$ große, auf der Rückseite abgedeckte Filmstücke in 10 ml 33° C warmes demineralisiertes Wasser gelegt, das leicht durchmischt, täglich gewechselt und analysiert wurde.

Abb. 1 beschreibt die Clonidin-Freigabe aus Polyacrylatfilmen als Funktion der Zeit. Aus dem Vergleich der Muster 159, 161 und 168 wird deutlich, in welchem Maße die Freigabe vom Wirkstoffprofil über den Filmquerschnitt abhängt. Der Vergleich der Muster 159/162 und 158/159 veranschaulicht darüber hinaus den Effekt des Lösungsmittels bzw. wasserbindender Zusatzstoffe (wie z. B. Polyäthylenglykol 400).

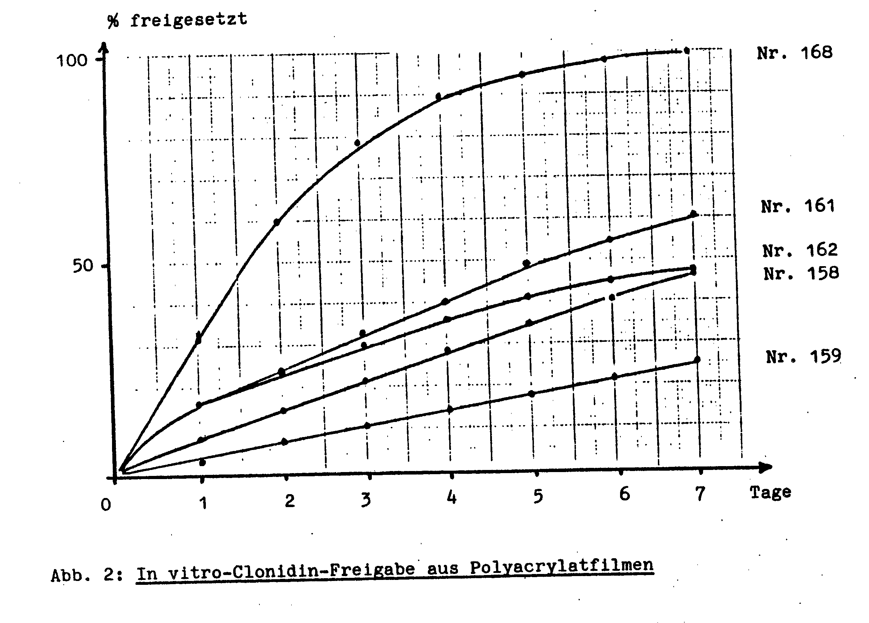

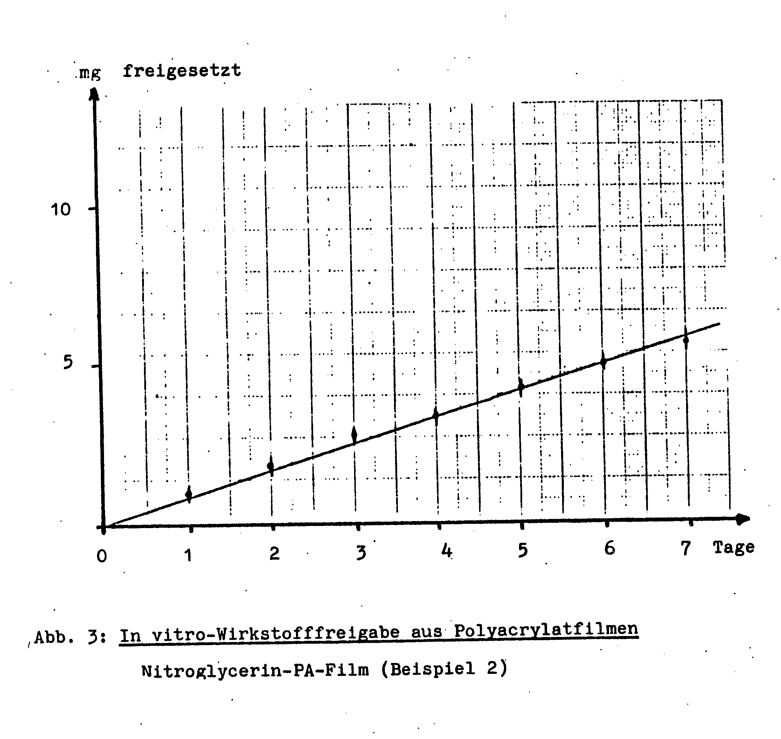

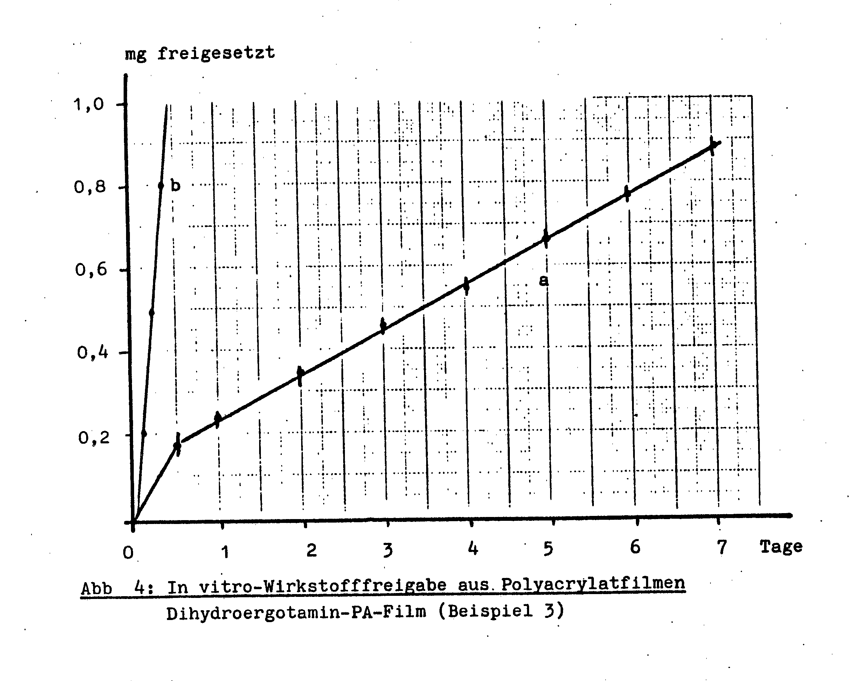

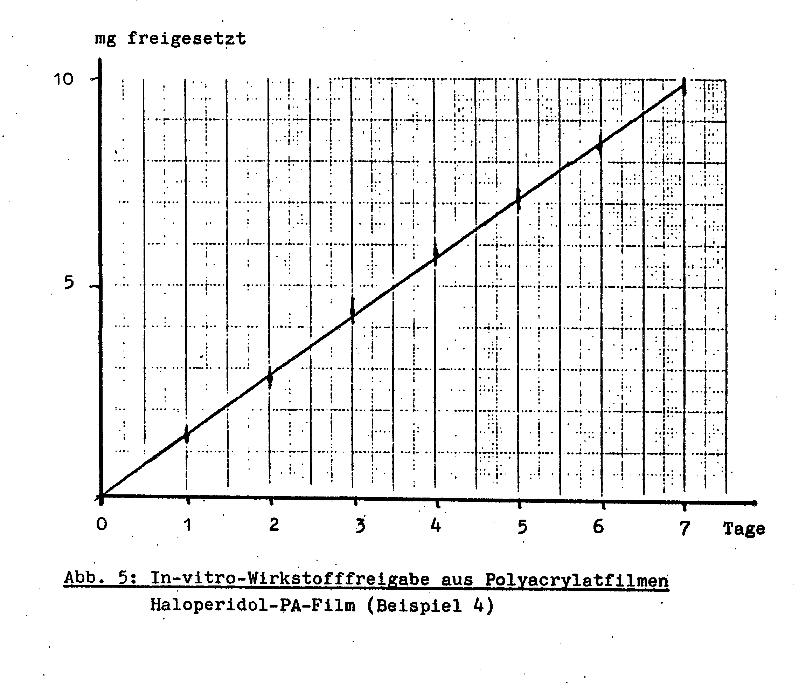

Abb. 2—5 enthalten die Freigabecharakteristiken der in den Beispielen 2, 3 und 4 beschriebenen wirkstoffhaltigen Polyacrylatfilme.

Die Temperaturabhängigkeit der Wirkstofffreigabe ist für eine konstante Dosierung von großer Bedeutung. Tabelle 2 enthält Daten, die zeigen, daß Polyacrylatfilme, wie z. B. das Muster 158, im Bereich 25—35° C in ihrem Freigabeverhalten nur wenig temperaturempfindlich sind.

Das Ad- und Desorptionsverhalten der Polyacrylatfilme ist für die Stabilität und die biopharmazeutische Qualität ein wichtiger Faktor. Die Wasseraufnahme der Filme bei 99%iger rel. Luftfeuchte wird z. B. durch Polyäthylenglykol 400, Glycerin und Sorbit stark erhöht (s. Tabelle 3).

**Tabelle 1**
Zusammensetzung verschiedener Clonidin-Polyacrylat-Filme

| Muster Nr. | Lösungsmittel | Wirkstoff aufgetragen %ig | ml/cm² | mg Poly-acrylat/ cm² |
|---|---|---|---|---|
| 124 | Methylenchlorid | 1,65 | 0,127 | 51,0[e]) |
| 137 | Methylenchlorid | 1,65 | 0,127 | 51,0[c]) |
| 138 | Methylenchlorid | 1,65 | 0,127 | 51,0[f]) |
| 139 | Methylenchlorid | 1,65 | 0,127 | 51,0[d]) |
| 158 | Äthanol | 3,3 | 0,0637 | 35,7[a]) |
| 159 | Äthanol | 3,3 | 0,0637 | 35,7[b]) |
| 161 | Äthanol | 3,3 | 0,0637 | 25,5 |
| 162 | Äthanol + $H_2O$ (2 + 1) | 3,3 | 0,0637 | 35,7[b]) |
| 168 | Äthanol | 3,3 | 0,0637 | 15,3 |

[a]) davon 10,2 mg Polyacrylat + 10% Polyäthylenglykol 400/cm² nachträglich aufgetragen
[b]) davon 10,2 mg Polyacrylat/cm² nachträglich auftragen
[c]) davon 25,5 mg Polyacrylat/cm² nachträglich auftragen
[d]) davon 25,5 mg Polyacrylat + 10% Karion F/cm² nachträglich auftragen
[e]) davon 25,5 mg Polyacrylat + 25% Polyäthylenglykol 400/cm² nachträglich auftragen
[f]) davon 25,5 mg Polyacrylat + 10% Glycerin/cm² nachträglich auftragen

**Tabelle 2**
Temperaturabhängigkeit der
in vitro-Clonidin-Freigabe aus
Polyacrylatfilmen

Muster Nr. 158

| Temperatur | % freigesetzt nach 0—1 d | 1—2 d | 2—3 d |
|---|---|---|---|
| 25° C | 7,0 | 6,0 | 6,0 |
| 33° C | 8,0 | 6,5 | 6,5 |

5

Tabelle 3
Ad- und Desorptionsisothermen von PA-Filmen mit verschiedenen Hilfsstoffzusätzen

Adsorption: 99% r.F. 37° C, anschließend
Desorption: 40% r.F. 23° C

| Muster | Hilfsstoff | Adsorption % H$_2$O (0−2 h) | % H$_2$O Max. (0−7 d) | Desorption % H$_2$O (0−2 h) | % H$_2$O Max. (0−2 d) |
|---|---|---|---|---|---|
| 137 | —. | 1,1 | 15 | 4,0 | 15 |
| 138 | 9% Glycerin | 2,6 | 50 | 5,0 | 50 |
| 139 | 9% Karion F | 5,0 | 40 | 6,0 | 40 |
| 124 | 25% PÄG 400 | 4,2 | 70 | 10,0 | 70 |

**Patentansprüche**

1. Pharmazeutische, für die transdermale Applikation von Wirkstoffen geeignete Zubereitung in Form eines Films aus einem Copolymerisat von Methyl- und/oder Äthylestern der Acryl- und Methacrylsäure, dadurch gekennzeichnet, daß der Wirkstoff im Polymerfilm in amorpher Form bzw. als feste Lösung in ansteigender Konzentration mit zunehmender Entfernung von der Freigabefläche vorliegt.

2. Verfahren zur Herstellung einer Zubereitung nach Anspruch 1, dadurch gekennzeichnet, daß man eine wäßrige Polyacrylat-Dispersion auf eine planare begrenzte Fläche ausgießt, sie zu einem Film trocknen läßt, eine organische Lösung oder eine Suspension des Arzneistoffes auf den trocknen Polyacrylatfilm aufträgt und das Lösungsmittel verdampft.

3. Verfahren nach Anspruch 2, dadurch gekennzeichnet, daß man der wäßrigen Polyacrylat-Dispersion hydrophile Hilfsstoffe und/oder gut wasserlösliche Arzneistoffe zusetzt.

4. Verfahren nach Anspruch 2 und/oder 3, dadurch gekennzeichnet, daß man auf den mit Wirkstoff beladenen Film erneut einmal oder mehrmals Polyacrylat-Dispersion aufträgt, diese trocknet und gegebenenfalls mit Wirkstoff belädt.

**Claims**

1. Pharmaceutical· preparation suitable for the transdermal application of active substances, in the form of a film consisting of a copolymer of methyl and so-called ethyl esters of acrylic and methacrylic acid, characterised in that the active substance is present in the polymer film in amorphous form or as a solid solution in a drawing concentration with an increase in distance from the release surplus.

2. Producing a preparation according to claim 1, characterised in that an aqueous polyacrylic dispersion is poured out on to a planar limited surface and allowed to dry into a film, an organic solution or a suspension of the medicament is applied to the dry polyacrylate film, and the solvent is evaporated.

3. Process according to claim 2, characterised in that hydrophilic excipient and/or highly water-soluble medicaments are added to the aqueous polyacrylate dispersion.

4. Process according to claim 2 and/or 3, characterised in that a polyacrylate dispersion is applied again once or several times to the film laden with active substance, is dried and, if appropriate, is laden with active substance.

**Revendications**

1. Préparation pharmaceutique adaptée à l'application transdermique de substances actives sous forme d'un film d'un copolymère d'esters métylique et/ou éthylique des acides acrylique et méthacrylique, caractérisée en ce que la substance active se trouve dans le film de polymère sous forme amorphe ou de solution solide en concentration croissante à mesure que l'éloignement par rapport à la surface de libération croît.

2. Procédé pour la fabrication d'une préparation selon la revendication 1, caractérisé en ce qu'on verse une dispersion aqueuse de polyacrylate sur une surface plane délimitée, on la laisse sécher pour

**0 020 905**

donner un film, on apporte une solution organique ou une suspension de la substance médicamenteuse sur le film de polyacrylate séché et on évapore le solvant.

3. Procédé selon la revendication 2, caractérisé en ce qu'on ajoute des adjuvants hydrophiles et/ou des substances médicamenteuses bien solubles dans l'eau à la dispersion aqueuse de polyacrylate.

4. Procédé selon la revendication 2 et/ou 3, caractérisé en ce qu'on apporte de nouveau une fois ou plusieurs fois de la dispersion de polyacrylate sur le film chargé avec de la substance active, sèche cette dispersion et éventuellement la charge avec de la substance active.

7

Abb. 1: Profil der Wirkstoffkonzentration C
im Filmquerschnitt

**Abb. 2:** <u>In vitro-Clonidin-Freigabe aus Polyacrylatfilmen</u>

**Abb. 3:** <u>In vitro-Wirkstofffreigabe aus Polyacrylatfilmen</u>

Nitroglycerin-PA-Film (Beispiel 2)

Abb 4: In vitro-Wirkstofffreigabe aus Polyacrylatfilmen
Dihydroergotamin-PA-Film (Beispiel 3)

Abb. 5: In-vitro-Wirkstofffreigabe aus Polyacrylatfilmen Haloperidol-PA-Film (Beispiel 4)